# FASCICULE DE BREVET EUROPEEN

(11) **EP 1 172 096 B1**
(45) Date de publication et mention de la délivrance du brevet: **25.09.2002**
(21) Numéro de dépôt: 01401834.5
(22) Date de dépôt: 09.07.2001
(51) Int. Cl.: A61K 7/50, A61K 7/02, A61K 7/00

(54) **Composition de nettoyage moussante sous la forme d'un gel transparent**
Schäumendes Reinigungsmittel in Form eines transparenten Gels
Foaming cleansing composition in the form of a transparent gel

(30) Priorité: 12.07.2000 FR 0009111
(43) Date de publication de la demande: 16.01.2002
(73) Titulaire: L'OREAL, 75008 Paris (FR)
(72) Inventeur: Guillou, Véronique, 92160 Antony (FR); Sebillotte-Arnaud Laurence, 94240 l'Hay-Les-Roses (FR); Bordeaux, Dominique, 91310 Longpont-sur-Orge (FR)
(74) Mandataire: Rasson, Catherine

(56) Documents cités:
- WO-A-94/17783
- DE-A- 19 937 917
- US-A- 4 381 259
- US-A- 5 653 970
- DATABASE WPI Week 199801 Derwent Publications Ltd., London, GB; AN 1988-231723 XP002168439 & JP 63 165477 A (LION CORP.)

## Description

L'invention a pour objet une composition de nettoyage moussante, rinçable à l'eau, et ayant l'aspect d'un gel transparent, comprenant un tensioactif du type phosphate, un tensioactif non ionique moussant et un polymère cationique, ainsi qu'à ses utilisations dans les domaines cosmétique ou dermatologique, notamment comme produit de nettoyage ou de démaquillage de la peau, du cuir chevelu et/ou des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

Pour le nettoyage de la peau, il est connu d'utiliser des gels aqueux détergents moussants. Leur action nettoyante est apportée par les tensioactifs qu'ils contiennent, ces tensioactifs mettant en suspension les résidus gras et les pigments des produits de maquillage. Ces gels sont efficaces et agréables à utiliser du fait qu'ils moussent. On cherche notamment à faire des gels nettoyants moussants transparents car, tout comme l'eau, la transparence est le symbole de pureté et donc de propreté, et les gels transparents sont ainsi particulièrement appréciés des utilisateurs. Les gels transparents moussants destinés au nettoyage du visage ou du corps génèrent très souvent des mousses aérées, légères. Toutefois, après rinçage, la peau est souvent glissante et il ne reste pas la sensation d'une peau propre du fait de la présence d'un résidu filmogène sur la peau, difficile à éliminer. Une façon d'obtenir des mousses denses, à fines bulles et se rinçant rapidement sans laisser de film consiste à utiliser des savons (sels d'acides gras) comme tensioactifs principaux. Toutefois, les compositions avec savon sont moins bien tolérées, particulièrement par les peaux sensibles. En outre, elles ne sont pas transparentes.

Il subsiste donc le besoin d'un gel aqueux moussant ne comportant pas de savon et ayant une bonne qualité de mousse tout en ayant une bonne qualité de rinçage et une bonne tolérance oculaire et cutanée.

La demanderesse a découvert de manière surprenante qu'on pouvait atteindre le but de l'invention et obtenir un gel transparent moussant ayant à la fois de bonnes propriétés cosmétiques (qualités de la mousse et qualité du rinçage) et de bonnes propriétés de tolérance, en utilisant l'association d'un tensioactif du type phosphate, d'un tensioactif non ionique moussant ayant des propriétés moussantes particulières et d'un polymère cationique exempt de groupes saccharides.

Certes, il est connu de préparer des compositions moussantes contenant des tensioactifs du type phosphate. Ainsi, le document GB-A-2,283,755 décrit l'association de MAP (monoalkylphosphate), d'un tensioactif non phosphaté et d'un polymère conditionneur de la peau pour préparer des produits de nettoyage moussant. Les tensioactifs non ioniques moussants ne sont pas décrits parmi les tensioactifs non phosphatés, et les compositions décrites dans ce document présentent l'inconvénient de donner une mousse ni assez dense, ni assez volumineuse.

Par ailleurs, le document EP-A-337354 décrit l'association d'alkylpolyglucosides (APG) et de polymères cationiques. Toutefois, les compositions décrites dans ce document présentent l'inconvénient de générer une mousse rêche et de laisser après rinçage un film sur la peau.

Ainsi, la présente demande a pour objet une composition de nettoyage, ayant l'aspect d'un gel transparent, contenant, dans un milieu aqueux physiologiquement acceptable, au moins un tensioactif du type phosphate, au moins un tensioactif non ionique moussant et au moins un polymère cationique exempt de groupes saccharides.

On entend ici par « milieu physiologiquement acceptable » un milieu compatible avec la peau, les muqueuses, le cuir chevelu, les yeux et/ou les cheveux. Par ailleurs, il s'agit d'un milieux aqueux, c'est-à-dire d'un milieu comportant une quantité d'eau d'au moins 35 % en poids, allant de préférence de 35 à 95 % en poids et mieux de 40 à 80 % en poids par rapport au poids total de la composition.

Le mot « transparent » signifie que la composition a une turbidité inférieure ou égale à 500 NTU. Les NTU (Nephelometric Turbidity Units) sont les unités de mesure de la turbidité d'une composition. La mesure de turbidité peut être faite par exemple avec un turbidimètre model 2100P de la société HACH Compagny, les tubes utilisés pour la mesure étant référencés AR397A cat 24347-06. Les mesures sont effectuées à température ambiante (20°C à 25°C). La transparence d'une composition peut se mesurer soit par le coefficient de transmittance à 600 nm soit par la turbidité. La composition de l'invention a un coefficient de transmittance à 600 nm allant de 10 à 90 % ou bien une turbidité allant de 2 à 500 NTU, et de préférence de 5 à 300 NTU.

La viscosité des compositions selon l'invention va de préférence de 0,1 à 200 poises (0,01 à 20 Pa.s), et mieux de 5 à 100 poises (0,5 à 10 Pa.s), mesurée à 25°C à l'aide du Rheomat RM180 de Rheometric Scientific, cet appareil étant équipé d'un mobile différent selon les viscosités, par exemple d'un mobile 2 pour les gammes de viscosités inférieures à 7 poises, d'un mobile 3 pour les gammes de viscosités de 2 à 40 poises, et d'un mobile 4 pour les gammes de viscosités de 20 poises à 80 poises.

L'association du tensioactif du type phosphate et du tensioactif non ionique moussant avec le polymère cationique ne comportant pas de groupe saccharide permet d'obtenir une mousse volumineuse avec de petites bulles, douce et dense. En revanche, si on remplace le polymère cationique ne comportant pas de groupe saccharide par un dérivé de polysaccharide cationique tel qu'un dérivé cationique de gomme guar ou de cellulose ou d'amidon ou de galactomannane, la composition obtenue laisse sur la peau, au moment du rinçage, un film glissant peu apprécié par les utilisateurs. En outre, les dérivés cationiques de gomme guar ont tendance à laisser un film collant.

Les compositions de l'invention ont l'avantage d'être très stables et de ne présenter ni déphasage ni phénomènes de recristallisation au stockage de 4°C à 45°C et même si elles sont soumises à des cycles -20°C/+20°C.

### 1. Tensioactif du type phosphate

Le tensioactif du type phosphate est choisi parmi les mono-alkylphosphates, les dialkylphosphates, leurs sels et leurs mélanges. Ces monoalkylphosphates et dialkyl phosphates comportent une ou plusieurs chaînes alkyle linéaires ou ramifiées, aliphatiques et/ou aromatiques, ayant de 8 à 22 atomes de carbone. Dans le mélange de mono- et di-alkylphosphates, la proportion mono/di peut aller de 100/0 à 50/50. Ces phosphates peuvent être neutralisés par des bases organiques ou minérales telles que la potasse, la soude, la triéthanolamine, l'arginine, la lysine et la N- methylglucamine.

Comme tensioactifs du type phosphate utilisables dans la composition de l'invention, on peut citer par exemple le mono-phosphate de lauryle tel que le produit commercialisé sous la dénomination MAP 20® par la société Kao Chemicals, le sel de potassium de l'acide dodécyl-phosphorique tel que le mélange de mono- et di-ester (diester majoritaire) commercialisé sous la dénomination CRAFOL AP-31® par la société Cognis, les monoester et di-ester d'acide octylphosphorique, tel que le mélange commercialisé sous la dénomination CRAFOL AP-20® par la société Cognis, les monoester et de diester d'acide phosphorique de 2-butyloctanol éthoxylé (7 moles d'OE), tel que le mélange commercialisé sous la dénomination ISOFOL 12 7 EO-PHOSPHATE ESTER® par la société Condea, les sels de potassium ou de triéthanolamine de mono-alkyl (C12-C13) phosphate, tels que le produit commercialisé sous les références ARLATONE MAP 230K-40® et ARLATONE MAP 230T-60® par la société Uniqema, le lauryl phosphate de potassium tel que le produit en solution aqueuse à 40 %, commercialisé sous la dénomination DERMALCARE MAP XC-99/09® par la société Rhodia Chimie, et les mélanges de ces tensioactifs.

La quantité de tensioactif(s) du type phosphate peut aller par exemple de 1 à 50 % en poids de matière active, et de préférence de 1,5 à 20 % en poids de matière active, par rapport au poids total de la composition.

### 2. Tensioactif non ionique

Les tensioactifs non ioniques utilisés dans la composition de l'invention sont des tensioactifs moussants, c'est-à-dire qu'ils génèrent une hauteur de mousse H telle décrite ci-dessous, égale ou supérieure à 3 cm et de préférence égale ou supérieure à 5 cm.

### Détermination de la hauteur de mousse d'un tensioactif

La détermination de la hauteur de mousse produite par un tensioactif est réalisée par caractérisation du comportement moussant sous agitation au vortex. On y observe:
◆ la cinétique de montée en mousse,
◆ la capacité à re-mousser après ajout d'eau,
◆ la stabilité dans le temps de la mousse formée.

La matériel utilisé est un agitateur vibrant de type vortex, HEIDOLPH, REAX 2000, équipé de tubes en verre à fond rond (hauteur 15 cm, diamètre 2 cm) avec des bouchons à vis, d'un portoir pour stocker ces tubes, d'un feutre indélébile et d'un double décimètre.

On prépare une solution aqueuse de tensioactif à 13 % de matière active dans l'eau permutée, ajustée à pH 7 avec une solution d'acide citrique ou de potasse.

Le test est réalisé de la manière suivante :
◆ on allume le VORTEX en position continue et à la vitesse maximale 9.
◆ on positionne le tube rempli de la solution sur le vortex et on le maintient par l'index, entre le pouce et le majeur, en appuyant verticalement, ceci pendant 15 secondes. Après l'arrêt de l'agitation, on met au feutre 2 traits sur le tube correspondant à la hauteur de mousse = hauteur au temps 1.
◆ On retourne une fois le tube en s'assurant que le liquide et la mousse se mélangent puis on le positionne à nouveau sur le vortex et on suit la même démarche que précédemment ; on laisse agiter 30 secondes et on note le niveau = hauteur au temps 2.
◆ On retourne une fois le tube puis on le positionne à nouveau sur le vortex et on suit la même démarche que précédemment ; on laisse agiter 1 minute et on note le niveau = hauteur au temps 3.
◆ Après cette dernière mesure, on ajoute dans le tube 7,0 g d'eau permutée ;
◆ on retourne 3 fois le tube et on réagite 30 secondes au VORTEX en suivant la même démarche, et on note le niveau = hauteur au temps 4 = hauteur H.
◆ on pose le tube sur le portoir et on note les niveaux après 5 min, 10 min, 20 min, 30 min et 1h de repos = hauteurs au temps 5 à 9.
◆ on mesure en cm la hauteur de mousse repérée par les traits sur le tube.

Les résultats sont exprimés sous forme de graphes de hauteurs de mousse en fonction du temps.

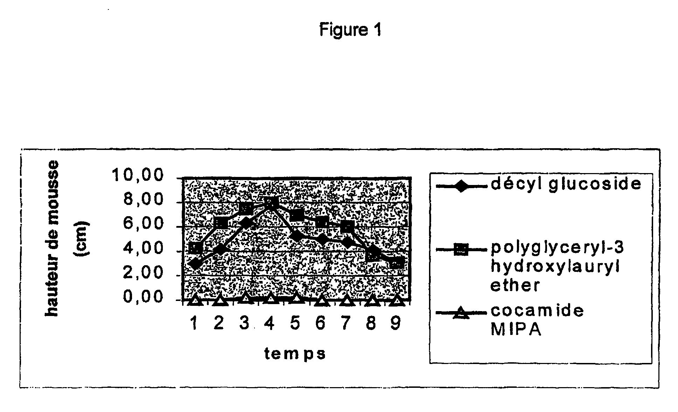

La figure 1 présente les hauteurs de mousse pour différents tensioactifs non ioniques. Le cocamide MIPA présente des hauteurs de mousse de 1 à 9 quasiment nulles alors que le décylglucoside et le polylgyceryl-3hydroxylauryl éther ont des hauteurs de mousse qui sont au départ d'environ 3 ou 4 cm, et ils ont une hauteur H (hauteur au temps 4) d'environ 8 cm, donc bien supérieure à 5 cm.

Les tensioactifs non ioniques utilisés selon l'invention ont une hauteur H de mousse de préférence supérieure à 3 cm, et notamment égale ou supérieure à 5 cm.

Comme tensioactifs non ioniques susceptibles d'être utilisés dans la composition de l'invention, on peut citer par exemple les alcools gras polyglycérolés ; les esters d'acides gras et de polyol ; les alkamides alkoxylés ; les dérivés de glucamine ; les alkylpolyglucosides ; et leurs mélanges.

Les alcools gras polyglycérolés sont des alkyl éthers de polyglycéryle, avec un groupe alkyle comportant de 8 à 30 atomes de carbone et de préférence de 10 à 22 atomes de carbone. Comme alcools gras polyglycérolés, on peut citer par exemple le dodecanediol polyglycérolé (3,5 moles de glycérol) ou Polyglyceryl-3 hydroxylauryl Ether (nom CTFA) vendu sous la dénomination CHIMEXANE NF par la Société Chimex.

Les esters d'acides gras et de polyol ont une chaîne grasse comportant de 8 à 30 atomes de carbone et de préférence de 10 à 22 atomes de carbone. Le polyol peut être notamment le glycérol ou les polymères de glycérol (polyglycérol) comportant plusieurs unités de glycérol. Comme esters d'acides gras et de polyol, on peut citer par exemple le monolaurate de polyglycérol tel que le produit commercialisé sous la dénomination SUNSOFT M-12J par la société Taiyo Kagaku.

Comme alkamides alkoxylés, on peut citer notamment les alkamides éthoxylés comme le PEG-5 COCAMIDE (nom CTFA) et en particulier le produit commercialisé sous la dénomination GENAGEN CA-050 par la société Clariant.

Comme les dérivés de N-alkylglucamine, on peut citer notamment l'éthyl-2 hexyl oxy-carbonyl N-méthyl glucamine.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de saccharide. Comme alkylpolyglucosides, on peut citer par exemple le decylglucoside (Alkyl-C9/C11-polyglucoside (1.4)) comme le produit commercialisé sous la dénomination MYDOL 10 par la société Kao Chemicals, le produit commercialisé sous la dénomination PLANTAREN 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel.

La quantité de tensioactif(s) non ionique(s) moussant dans la composition de l'invention peut aller par exemple de 1 à 50 % en poids de matière active, de préférence de 1,5 à 20 % en poids de matière active, par rapport au poids total de la composition.

### 3. Polymère cationique exempt de groupe saccharide

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques utilisables dans la composition de l'invention, on peut citer plus particulièrement les polymères suivants et leurs mélanges :

(1) Les homopolymères ou copolymères d'esters ou d'amides acryliques ou méthacryliques, comportant un anion dérivé d'un acide minéral ou organique tel qu'un anion méthosulfate ou un halogénure tel que chlorure ou bromure.

Comme copolymères de la famille (1), on peut citer par exemple :
- les copolymères d'acrylamide et de sel de méthacrylyloxyéthyltriméthyl-ammonium, comme le copolymère d'acrylamide et de chlorure de bêtaméthacrylyloxyéthyltriméthyl-ammonium (nom CTFA : Polyquaternium 15) tel que le produit commercialisé sous la dénomination ROHAGIT KF 720 F par la société Rohm, et comme le copolymère d'acrylamide et de méthosulfate de bêtaméthacrylyloxyéthyltriméthyl-ammonium (nom CTFA : Polyquaternium 5) tel que le produit commercialisé sous la dénomination MERQUAT 5 par la société Calgon ;
- les copolymères vinylpyrrolidone / acrylate ou méthacrylate de dialkylaminoalkyle quaternisés ou non, comme le polymère ammonium quaternaire formé par récation de diéthylsulfate et d'un copolymère de vinyl-pyrrolidone et de diméthylaminoéthylméthacrylate (nom CTFA: Polyquatemium 11) tel que les produits commercialisés sous les dénominations GAFQUAT 755, GAFQUAT 755N et GAFQUAT 734 par la société ISP ;
- les copolymères acide acrylique / méthylacrylate / chlorure de méthacrylamidopropyltrimonium (nom CTFA : Polyquaternium 47) tel que le produit commercialisé sous la dénomination MERQUAT 2001N par la société Calgon.

(2) Les polymères d'alkyl diallyl amine ou de dialkyl diallyl ammonium, en particulier les homopolymères de sel de diméthyldiallylammonium et les copolymères de sel de diméthyldiallylammonium et d'acrylamide ou d'acide acrylique ou méthacrylique.

Comme polymères de la famille (2), on peut citer en particulier l'homopolymère de chlorure de diméthyldiallylammonium (nom CTFA : Polyquaternium 6) tel que le produit commercialisé sous la dénomination SALCARE SC 30 par la société CIBA et le produit commercialisé sous la dénomination MERQUAT 100 par la société Calgon ; le copolymère de chlorure de diméthyldiallylammonium et d'acrylamide (nom CTFA : Polyquatemium 7) tel que les produits commercialisés sous les dénominations MERQUAT S, MERQUAT 2200 et MERQUAT 550 par la société Calgon et le produit commercialisé sous la dénomination SALCARE SC 10 par la société Ciba ; le copolymère de chlorure de diméthyldiallylammonium et d'acide acrylique (nom CTFA: Polyquatemium 22) tel que le produit commercialisé sous la dénomination MERQUAT 280 par la société Calgon ; le sel d'ammonium polymérique d'acide acrylique, de chlorure de diallyldiméthylammonium et d'acrylamide (nom CTFA: Polyquaternium 39) tel que le produit commercialisé sous la dénomination MERQUAT PLUS 3330 ou 3331 par la société Calgon.

(3) Les polymères quaternaires de vinylpyrrolidone et d'imidazole ou de vinylimidazole ou de méthylvinylimidazole, comme le sel d'ammonium quaternaire polymérique formé de chlorure de méthylvinylimidazolium et de vinylpyrrolidone (nom CTFA : Polyquatemium 16) tel que les produits commercialisés sous les dénominations LUVIQUAT FC905, LUVIQUAT FC370, LUVIQUAT HM552 et LUVIQUAT FC550 par la société BASF; le sel d'ammonium quaternaire polymérique formé de vinylpyrrolidone et d'imidazoline quaternisé (nom CTFA : Polyquaternium 44) tel que les produits commercialisés sous les dénominations LUVIQUAT CARE et LUVIQUAT MS-370 par la société BASF ; le sel d'ammonium quaternaire polymérique formé par réaction de vinylcaprolactame et de vinylpyrrolidone avec le méthosulfate de méthylvinylimidazolium (nom CTFA : Polyquaternium 46) tels que le produit commercialisé sous la dénomination LUVIQUAT HOLD par la société BASF ;

(4) Les polymères de vinylpyrrolidone comportant des motifs méthacrylamidopropyldiméthylamine ou méthacrylamidopropyltriméthylammonium (M.A.P.T.A.C.) (nom CTFA : Polyquaternium 28), parmi lesquels on peut citer notamment les produits commercialisés sous les dénominations GAFQUAT HS-100 et STYLEZE CC-10 par la société I.S.P.

Parmi les polymères cationiques susceptibles d'être utilisés dans le cadre de la présente invention, on préfère mettre en oeuvre les copolymères d'acrylamide et de sel de méthacrylyloxyéthyltriméthyl-ammonium, notamment le Polyquaternium 5 ; le copolymère acide acrylique / méthylacrylate / chlorure de méthacrylamidopropyltrimonium (Polyquaternium 47); les copolymères de chlorure de diméthyldiallylammonium, notamment le Polyquatemium 7 et le Polyquaternium 39; le polymère de vinylpyrrolidone comportant des motifs méthacrylamidopropyldiméthylamine ou méthacrylamidopropyltriméthylammonium (Polyquaternium 28); les polymères quaternaires de vinylpyrrolidone et d'imidazole, notamment le Polyquaternium 44 ; et les mélanges de ces polymères.

Ces polymères cationiques, en association avec le tensioactif de type phosphate et le tensioactif non ionique moussant, apportent de la douceur à la mousse, diminuent la taille des bulles en général et procurent un rinçage tel que recherché.

La quantité de polymère(s) cationique(s) peut aller par exemple de 0,01 à 5 % en poids de matière active et de préférence de 0,05 à 2 % en poids de matière active par rapport au poids total de la composition.

Selon un mode préféré de réalisation de l'invention, la composition comprend (1) au moins un tensioactif du type phosphate choisi parmi le mono-phosphate de lauryle, le sel de potassium de l'acide dodécyl-phosphorique, les monoester et diester d'acide octylphosphorique, les monoester et diester d'acide phosphorique de 2-butyloctanol éthoxylé (7 moles d'OE), les sels de potassium ou de triéthanolamine de mono-alkyl (C12-C13) phosphate, le lauryl phosphate de potassium, (2) au moins un tensioactif non ionique moussant choisi parmi les alkylpolyglucosides et les alcools gras polyglycérolés et (3) au moins un polymère cationique choisi parmi le polyquaternium 5, le Polyquaternium 47, le Polyquaternium 7, le Polyquaternium 39, le Polyquaternium 28 et le Polyquaternium 44.

Selon un mode particulier de réalisation de l'invention, la composition selon l'invention comprend du lauryl phosphate de potassium, du décyl glucoside et/ou du Polyglyceryl-3 hydroxylauryl Ether, et du Polyquaternium-7.

Le milieu aqueux des compositions moussantes de l'invention peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition.

Pour obtenir des compositions plus ou moins fluides, on peut incorporer dans les compositions de l'invention un ou plusieurs agents épaississants, notamment des polymères, dans des concentrations allant par exemple de 0,05 à 10 % en poids de matière active, de préférence de 0,2 à 5 % en poids et mieux de 0,2 à 2 % en poids par rapport au poids total de la composition.

On peut citer comme exemples d'épaississants, les sels minéraux tels que le chlorure de sodium ; les molécules oxyéthylénées et notamment les dérivés alkyl ou acyl éthoxylés de polyols qui peuvent être en particulier des dérivés oxyéthylénés d'esters d'acides gras ou d'éthers d'alcools gras et de polyols tel que glycérol, sorbitol, glucose, pentaerythritol. Comme composés de ce type, on peut citer par exemple le stéarate de glycéryle oxyéthyléné (200 OE) tel que le produit commercialisé sous la dénomination SIMULSOL 220 TM ® par la société Seppic, le tétrastéarate de pentaérythrityle oxyéthyléné (150 OE) tel que le produit commercialisé sous la dénomination CROTHIX ® par la société Croda, le dioléate de méthylglucose oxyéthyléné (120 OE) tel que le produit commercialisé sous la dénomination GLUCAMATE DOE-120 VEGETAL® par la société Amerchol, le triisostéarate de sorbitan oxyéthyléné (160 OE) tel que le produit commercialisé sous la dénomination RHEODOL TW IS399C par la société Kao Chemicals.

En outre, les compositions de l'invention peuvent contenir des adjuvants habituellement utilisés dans le domaine cosmétique, choisis parmi les parfums, les conservateurs, les séquestrants (EDTA), les pigments, les nacres, les charges minérales ou organiques, les agents pour ajuster le pH, les colorants solubles, les filtres solaires, les actifs. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Les compositions selon l'invention ont l'apparence d'un gel transparent. En outre, ces compositions sont stables et ont une très bonne rinçabilité. Elles peuvent être utilisées en particulier dans les domaines cosmétique ou dermatologique, et constituer par exemple un produit de nettoyage et/ou de démaquillage de la peau, du cuir chevelu et/ou des cheveux, un produit de gommage et/ou un produit exfoliant pour la peau. Elles peuvent constituer plus particulièrement une composition de nettoyage de la peau.

Un autre objet de l'invention consiste en l'utilisation cosmétique de la composition telle que définie ci-dessus, comme produits de nettoyage et/ou de démaquillage de la peau, du cuir chevelu et/ou des cheveux, et/ou comme produit de gommage et/ou produit exfoliant pour la peau.

Les compositions selon l'invention peuvent être utilisés de deux façons:
- la première utilisation consiste à étaler le gel dans les mains, à l'appliquer sur le visage ou sur le corps puis à le masser en présence d'eau pour développer la mousse directement sur le visage ou le corps.
- l'autre utilisation possible de ce type de produit consiste à développer la mousse dans les paumes des mains avant de l'appliquer sur le visage ou le corps.

Dans les deux cas, la mousse est ensuite rincée.

Un autre objet de l'invention consiste en un procédé cosmétique de nettoyage de la peau, du cuir chevelu et/ou des cheveux, caractérisé par le fait qu'on applique la composition de l'invention, sur la peau, sur le cuir chevelu et/ou sur les cheveux, en présence d'eau, et qu'on élimine la mousse formée et les résidus de salissure par rinçage à l'eau.

Les compositions selon l'invention peuvent constituer aussi une composition pour le traitement des peaux grasse, notamment quand elles contiennent un actif spécifique de traitement des peaux grasses tels que, par exemple, l'acide salicylique, l'acide azélaïque, le triclosan, le piroctone olamine, la niacinamide (vitamine PP).

Un autre objet de l'invention est l'utilisation de la composition telle que définie ci-dessus pour la préparation d'une composition destinée à traiter la peau grasse.

Les exemples qui suivent servent à illustrer l'invention sans toutefois présenter un caractère limitatif. Les quantités indiquées sont en % en poids sauf mention contraire.

Dans le tableau ci-dessous, tous les pourcentages sont exprimés en poids de matières premières telles que commercialisées (et non en poids de matière active M.A.).

| Composition | Ex. 1 comparatif | Ex. 2 comparatif | Ex. 3 comparatif | Ex. 1 selon l'invention | Ex. 2 selon l'invention | Ex. 3 selon l'invention | Ex. 4 comparatif |
|---|---|---|---|---|---|---|---|
| Lauryl phosphate (1) | 13% | - | 6,5% | 6,5% | 3,35% | 6,5% | 6,5% |
| Decyl glucoside (2) | - | 32,5 % (M.A.= 13%) | 16,25% (M.A.= 6,5%) | 16.25% (M.A.= 6,5%) | 26,25% (M.A.= 10,5%) | - | - |
| Polyglyceryl -3 hydroxylauryl Ether (4) | - | - | - | - | - | 6,5% | - |
| Cocamide MIPA | - | - | - | - | - | - | 6,5% |
| Polyquaternium-7 (3) | - | - | - | 5,7 % | 5,7% | 2,3% | 2,3% |
| PEG-150 pentaérythri -tyl tétrastéarate | - | - | - | 0,5% | 0,5% | - | 0,5% |
| Monostéarate de glycéryle oxyéthyléné (200 OE) (5) | - | - | - | - | - | 0,5 % | - |
| Glycérine | 3,5% | 3,5% | 3,5% | 3,5% | 3,5% | 3,5% | 3,5% |
| Sorbitol | 3,5% | 3,5% | 3,5% | 3,5% | 3,5% | 3,5% | 3,5% |
| Hydroxyde de potassium | 3% | - | 1,7% | 1,7% | 0,9% | 1,7% | 1,7% |
| Hydroxypropyl-cellulose | - | - | - | 0,2 % | 0,2 % | 0,2 % | 0,2% |
| Disodium EDTA | 0,05 % | 0,05 % | 0,05% | 0,05% | 0,05% | 0,05% | 0,05% |
| Chlorure de sodium | 0,1% | 0,1% | 0,1% | 0,1% | 0,1% | 0,1% | 0,1% |
| Conservateurs | 0,3% | 0,3% | 0,3% | 0,3% | 0,3% | 0,3% | 0,3% |
| Eau | qsp 100 % | qsp 100 % | qsp 100 % | qsp 100 % | qsp 100 % | qsp 100 % | qsp 100 % |
| Aspect | solution | solution | solution | gel transparent | gel transparent | gel transparent | gel opaque instable |

(1) Mono-phosphate de lauryle (à 75 % de mono-ester) : MAP 20® de Kao Chemicals ;
(2) Alkyl (C9/11) Polyglucoside (1.4) en solution à 40% : MYDOL 10® de Kao Chemicals;
(3) Copolymere chlorure de di-methyl di-allyl ammonium / acrylamide à 8 % dans l'eau : MERQUAT S® de Calgon ;
(4) Dodecanediol polyglycérolé (3,5 moles) : Chimexane NF® de Chimex ;
(5) Tétra-stéarate de pentaérythrityle oxyéthyléné (150 OE) : CROTHIX® de Croda.

Performances sensorielles : les qualités de mousse développées sont évaluées selon le protocole décrit ci-dessous.

Avant toute utilisation du produit, les mains sont lavées au savon de Marseille puis convenablement rincées et séchées. Puis le protocole suivi est le suivant :
1- mouiller les mains en les passant sous l'eau courante, les secouer trois fois pour les essorer,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- rincer les mains sous l'eau ,
6- les essuyer.

Les critères sont évalués à chaque étape du protocole suivi, et ils sont notés sur une échelle de 0 à 10.
- étape 4 : évaluation de la qualité de mousse
- *Le volume de mousse:* la note attribuée est d'autant plus élevée que le volume est grand.
- *La taille* des *bulles composant la mousse :* la note attribuée est d'autant plus élevée que les bulles sont grosses.
- *La densité :* consistance, tenue de la mousse : la note attribuée est d'autant plus élevée que la densité est grande.
- *La douceur de la mousse :* la note attribuée est d'autant plus élevée que la mousse est douce.
- étape 5 : évaluation pendant le rinçage
- *Le rinçage :* la note attribuée est d'autant plus faible que la présence d'un film glissant difficile à éliminer est grande, ce qui signifie que la note est d'autant plus forte que le produit se rince facilement sans laisser de film.

Turbidité : elle est mesurée comme indiqué ci-dessus avec un turbidimètre model 2100P de la société HACH Compagny.

Les résultats sensoriels pour chacun des critères et la turbidité sont les suivants :

| Critères sensoriels | Ex.1 comparatif | Ex.2 comparatif | Ex.3 comparatif | Ex.1 de l'invention | Ex.2 de l'invention | Ex.3 de l'invention | Ex.4 comparatif |
|---|---|---|---|---|---|---|---|
| Volume de mousse | **4,6** | 7,5 | 6,5 | 5,1 | 5,2 | 5,4 | 5,7 |
| Taille des bulles | 3,25 | **7,4** | 4,75 | 3,5 | 4,5 | 3,5 | **5,9** |
| Densité de la mousse | **5,25** | 7,25 | 7,1 | 8,4 | 7 | 7,5 | 6,1 |
| Douceur de la mousse | **6,5** | **4** | **6,6** | 8,75 | 8,6 | 7,9 | 7,4 |
| Rinçage | 8,6 | 10 | 9 | 8,6 | 8,6 | 8 | 8,6 |
| Turbidité (NTU) | 22 | 15 | 52 | 26,5 | 14,3 | 12 | > 1000 |

Il ressort du tableau ci-dessus que l'exemple comparatif 1 qui ne comporte qu'un tensioactif phosphate donne un volume de mousse et une densité insuffisantes ainsi qu'une douceur moyenne, que l'exemple comparatif 2 qui ne comporte qu'un APG donne une taille de bulles trop élevée (mousse pas fine) et une douceur insatisfaisante, que l'exemple comparatif 3 qui ne comporte que l'association de phosphate et APG donne une douceur peu élevée, et que l'exemple comparatif 4 qui comporte un tensioactif ayant une hauteur de mousse H insuffisante donne une taille de bulles trop élevée (donc mousse peu fine) et c'est un gel opaque qui, en outre, est instable dans le temps.

En revanche, l'association selon l'invention permet d'obtenir à la fois un gel transparent, donnant une mousse fine, dense et aussi très douce, tout en conservant un bon volume de mousse et un bon rinçage.

## Revendications

1. Composition de nettoyage, ayant l'aspect d'un gel transparent et contenant, dans un milieu aqueux, au moins un tensioactif du type phosphate, au moins un tensioactif non ionique moussant et au moins un polymère cationique exempt de groupes saccharides.

2. Composition selon la revendication 1, **caractérisée en ce qu'**elle a une turbidité allant de 2 à 500 NTU.

3. Composition selon la revendication 1 ou 2, **caractérisée en ce que** le tensioactif du type phosphate est choisi parmi les mono-alkylphosphates, les dialkylphosphates, leurs sels et leurs mélanges.

4. Composition selon la revendication précédente, **caractérisée en ce que** le tensioactif du type phosphate est choisi parmi le mono-phosphate de lauryle, le sel de potassium de l'acide dodécyl-phosphorique, le mélange de monoester et de di-ester d'acide octylphosphorique, le mélange de monoester et de diester d'acide phosphorique de 2-butyloctanol éthoxylé (7 moles d'OE), le sel de potassium ou de triéthanolamine de mono-alkyl (C12-C13) phosphate, le lauryl phosphate de potassium en solution aqueuse à 40 %, et leurs mélanges.

5. Composition selon l'une quelconque des revendications précédentes, **caractérisée en ce que** la quantité de tensioactif(s) du type phosphate va de 1 à 50 % en poids de matière active, et de préférence de 1,5 à 20 % en poids de matière active, par rapport au poids total de la composition.

6. Composition selon la revendication précédente, **caractérisée en ce que** le tensioactif non ionique moussant donne une hauteur de mousse H égale ou supérieure à 3 cm et de préférence égale ou supérieure à 5 cm.

7. Composition selon l'une quelconque des revendications précédentes, **caractérisée en ce que** le tensioactif non ionique moussant est choisi parmi les alcools gras polyglycérolés ; les esters d'acides gras et de polyol ; les alkamides alkoxylés ; les dérivés de glucamine ; les alkylpolyglucosides ; et leurs mélanges.

8. Composition selon l'une quelconque des revendications précédentes, **caractérisée en ce que** la quantité de tensioactif(s) non ionique(s) va de 1 à 50 % en poids de matière active, et de préférence de 1,5 à 20 % en poids de matière active, par rapport au poids total de la composition.

9. Composition selon l'une quelconque des revendications précédentes, **caractérisée en ce que** le polymère cationique est choisi parmi les homopolymères ou copolymères d'esters ou d'amides acryliques ou méthacryliques, comportant un anion dérivé d'un acide minéral ou organique ; les polymères d'alkyl diallyl amine ou de dialkyl diallyl ammonium ; les polymères quaternaires de vinylpyrrolidone et d'imidazole ou de vinylimidazole ou de méthylvinylimidazole ; les polymères de vinylpyrrolidone comportant des motifs méthacrylamidopropyldiméthylamine ou méthacrylamidopropyltriméthylammonium ; et leurs mélanges.

10. Composition selon l'une quelconque des revendications précédentes, **caractérisée en ce que** le polymère cationique est choisi parmi le Polyquatemium 5, le Polyquaternium 6, le Polyquatemium 7, le Polyquaternium 11, le Polyquatemium 15, le Polyquatemium 16, le Polyquatemium 22, le Polyquaternium 28, le Polyquaternium 39, le Polyquatemium 44, le Polyquaternium 46, le Polyquatemium 47 et leurs mélanges.

11. Composition selon l'une quelconque des revendications précédentes, **caractérisée en ce que** la quantité de polymère(s) cationique(s) va de 0,01 à 5 % en poids de matière active et de préférence de 0,05 à 2 % en poids de matière active par rapport au poids total de la composition.

12. Composition selon l'une quelconque des revendications précédentes, **caractérisée en ce qu'**elle comprend (1) au moins un tensioactif du type phosphate choisi parmi le mono-phosphate de lauryle, le sel de potassium de l'acide dodécyl-phosphorique, les monoester et di-ester d'acide octylphosphorique, les monoester et diester d'acide phosphorique de 2-butyloctanol éthoxylé (7 moles d'OE), les sels de potassium ou de triéthanolamine de mono-alkyl (C12-C13) phosphate, le lauryl phosphate de potassium, (2) au moins un tensioactif non ionique moussant choisi parmi les alkylpolyglucosides et les alcools gras polyglycérolés et (3) au moins un polymère cationique choisi parmi le Polyquatemium 5, le Polyquatemium 47, le Polyquatemium 7, le Polyquatemium 39, le Polyquatemium 28 et le Polyquatemium 44.

13. Composition selon l'une quelconque des revendications précédentes, **caractérisée en ce qu'**elle comprend au moins un solvant choisi parmi les alcools inférieurs ; les polyols ; les sucres et leurs mélanges.

14. Composition selon l'une quelconque des revendications précédentes, **caractérisée en ce qu'**elle comprend au moins un agent épaississant.

15. Utilisation cosmétique d'une composition selon l'une quelconque des revendications 1 à 14, comme produits de nettoyage et/ou de démaquillage de la peau, du cuir chevelu et/ou des cheveux, et/ou comme produit de gommage et/ou produit exfoliant pour la peau.

16. Procédé cosmétique de nettoyage de la peau, du cuir chevelu et/ou des cheveux, **caractérisé en ce qu'**on applique la composition selon l'une quelconque des revendications 1 à 14, sur la peau, sur le cuir chevelu et/ou sur les cheveux, en présence d'eau, et qu'on élimine la mousse formée et les résidus de salissure par rinçage à l'eau.

17. Utilisation de la composition selon l'une quelconque des revendications 1 à 14, pour la préparation d'une composition destinée à traiter la peau grasse.

## Claims

1. Cleansing composition which has the appearance of a transparent gel and which comprises, in an aqueous medium, at least one surfactant of the phosphate type, at least one foaming nonionic surfactant and at least one cationic polymer devoid of saccharide groups.

2. Composition according to Claim 1, **characterized in that** it has a turbidity ranging from 2 to 500 NTU.

3. Composition according to Claim 1 or 2, **characterized in that** the surfactant of the phosphate type is chosen from monoalkyl phosphates, dialkyl phosphates, their salts and their mixtures.

4. Composition according to the preceding claim, **characterized in that** the surfactant of the phosphate type is chosen from monolauryl phosphate, the potassium salt of dodecyl phosphate, the mixture of octyl monoester and of octyl diester of phosphoric acid, the mixture of ethoxylated (7 mol of EO) 2-butyloctanol monoester and of ethoxylated (7 mol of EO) 2-butyloctanol diester of phosphoric acid, the potassium or triethanolamine salt of monoalkyl (C₁₂-C₁₃) phosphate, potassium lauryl phosphate as a 40% aqueous solution, and their mixtures.

5. Composition according to any one of the preceding claims, **characterized in that** the amount of surfactant(s) of the phosphate type ranges from 1 to 50% by weight of active material and preferably from 1.5 to 20% by weight of active material with respect to the total weight of the composition.

6. Composition according to the preceding claim, **characterized in that** the foaming nonionic surfactant gives a foam height H of equal to or greater than 3 cm and preferably of equal to or greater than 5 cm.

7. Composition according to any one of the preceding claims, **characterized in that** the foaming nonionic surfactant is chosen from polyglycerolated fatty alcohols; esters of fatty acids and of polyol; alkoxylated alkamides; glucamine derivatives; alkylpolyglucosides; and their mixtures.

8. Composition according to any one of the preceding claims, **characterized in that** the amount of nonionic surfactant(s) ranges from 1 to 50% by weight of active material and preferably from 1.5 to 20% by weight of active material with respect to the total weight of the composition.

9. Composition according to any one of the preceding claims, **characterized in that** the cationic polymer is chosen from homopolymers or copolymers of acrylic or methacrylic esters or amides comprising an anion derived from an inorganic or organic acid; polymers of alkyldiallylamine or of dialkyldiallylammonium; quaternary polymers of vinylpyrrolidone and of imidazole or of vinylimidazole or of methylvinylimidazole; vinylpyrrolidone polymers comprising methacrylamidopropyldimethylamine or methacrylamidopropyltrimethylammonium units; and their blends.

10. Composition according to any one of the preceding claims, **characterized in that** the cationic polymer is chosen from polyquaternium-5, polyquaternium-6, polyquaternium-7, polyquaternium-11, polyquaternium-15, polyquaternium-16, polyquaternium-22, polyquaternium-28, polyquaternium-39, polyquaternium-44, polyquaternium-46, polyquaternium-47 and their blends.

11. Composition according to any one of the preceding claims, **characterized in that** the amount of cationic polymer(s) ranges from 0.01 to 5% by weight of active material and preferably from 0.05 to 2% by weight of active material with respect to the total weight of the composition.

12. Composition according to any one of the preceding claims, **characterized in that** it comprises (1) at least one surfactant of the phosphate type chosen from monolauryl phosphate, the potassium salt of dodecyl phosphate, the octyl monoester and octyl diester of phosphoric acid, the ethoxylated (7 mol of EO) 2-butyloctanol monoester and the ethoxylated (7 mol of EO) 2-butyloctanol diester of phosphoric acid, the potassium or triethanolamine salts of monoalkyl (C₁₂-C₁₃) phosphate, or potassium lauryl phosphate, (2) at least one foaming nonionic surfactant chosen from alkylpolyglucosides and polyglycerolated fatty alcohols, and (3) at least one cationic polymer chosen from polyquaternium-5, polyquaternium-47, polyquaternium-7, polyquaternium-39, polyquaternium-28 and polyquaternium-44.

13. Composition according to any one of the preceding claims, **characterized in that** it comprises at least one solvent chosen from lower alcohols, polyols, sugars and their mixtures.

14. Composition according to any one of the preceding claims, **characterized in that** it comprises at least one thickening agent.

15. Cosmetic use of a composition according to any one of Claims 1 to 14 as products for cleansing and/or removing make-up from the skin, scalp and/or hair and/or as a scrub and/or exfoliant for the skin.

16. Cosmetic process for cleansing the skin, scalp and/or hair, **characterized in that** the composition according to any one of Claims 1 to 14 is applied to the skin, to the scalp and/or to the hair in the presence of water and **in that** the foam formed and the grime are removed by rinsing with water.

17. Use of the composition according to any one of Claims 1 to 14 in the preparation of a composition intended for the treatment of greasy skin.

## Patentansprüche

1. Zusammensetzung zur Reinigung, die das Aussehen eines transparenten Gels hat und die in einem wäßrigen Medium mindestens einen grenzflächenaktiven Stoff vom Phosphattyp, mindestens einen schäumenden nichtionischen grenzflächenaktiven Stoff und mindestens ein kationisches Polymer ohne Saccharidgruppen enthält.

2. Zusammensetzung nach Anspruch 1, **dadurch gekennzeichnet, daß** sie eine Trübung im Bereich von 2 bis 500 NTU aufweist.

3. Zusammensetzung nach Anspruch 1 oder 2, **dadurch gekennzeichnet, daß** der grenzflächenaktive Stoff vom Phosphattyp unter den Monoalkylphosphaten, Dialkylphosphaten, ihren Salzen und ihren Gemischen ausgewählt ist.

4. Zusammensetzung nach dem vorhergehenden Anspruch, **dadurch gekennzeichnet, daß** der grenzflächenaktive Stoff vom Phosphattyp unter Laurylmonophosphat, dem Kaliumsalz von Dodecylphosphorsäure, dem Gemisch des Monoesters und des Diesters von Octylphosphorsäure, dem Gemisch des Phosphorsäuremonoesters und des Phosphorsäurediesters von ethoxyliertem 2-Butyloctanol (7 mol EO), dem Kaliumsalz oder dem Triethanolaminsalz von Mono-C₁₂₋₁₃-Alkylphosphat, Kaliumlaurylphosphat in wäßriger Lösung von 40 % und deren Gemischen ausgewählt ist.

5. Zusammensetzung nach einem der vorhergehenden Ansprüche, **dadurch gekennzeichnet, daß** der Mengenanteil des (der) grenzflächenaktiven Stoffe(s) vom Phosphattyp im Bereich von 1 bis 50 Gew.-% Wirkstoff und vorzugsweise 1,5 bis 20 Gew.-% Wirkstoff, bezogen auf das Gesamtgewicht der Zusammensetzung, liegt.

6. Zusammensetzung nach dem vorhergehenden Anspruch, **dadurch gekennzeichnet, daß** der schäumende nichtionische grenzflächenaktive Stoff eine Schaumhöhe H von 3 cm oder darüber und vorzugsweise mindestens 5 cm ergibt.

7. Zusammensetzung nach einem der vorhergehenden Ansprüche, **dadurch gekennzeichnet, daß** der schäumende nichtionische grenzflächenaktive Stoff unter den mehrfach mit Glycerin veretherten Fettalkoholen, den Estern von Fettsäuren und Polyolen; alkoxylierten Alkamiden, Glucaminderivaten, Alkylpolyglucosiden und deren Gemischen ausgewählt ist.

8. Zusammensetzung nach einem der vorhergehenden Ansprüche, **dadurch gekennzeichnet, daß** der Mengenanteil des (der) nichtionischen grenzflächenaktiven Stoffe(s) im Bereich von 1 bis 50 Gew.-% Wirkstoff und vorzugsweise 1,5 bis 20 Gew.-% Wirkstoff, bezogen auf das Gesamtgewicht der Zusammensetzung, liegt.

9. Zusammensetzung nach einem der vorhergehenden Ansprüche, **dadurch gekennzeichnet, daß** das kationische Polymer unter den Homopolymeren oder Copolymeren von Acrylestern, Methacrylestern, Acrylamiden oder Methacrylamiden, die ein Anion enthalten, das von einer anorganischen oder organischen Säure abgeleitet ist; den Alkyldiallylaminpolymeren oder Dialkyldiallylammoniumpolymeren; den quartären Polymeren von Vinylpyrrolidon und Imidazol oder Vinylimidazol oder Methylvinylimidazol; den Polymeren von Vinylpyrrolidon, die Methacrylamidopropyldimethylamineinheiten oder Methacrylamidopropyltrimethylammoniumeinheiten aufweisen, und deren Gemischen ausgewählt ist.

10. Zusammensetzung nach einem der vorhergehenden Ansprüche, **dadurch gekennzeichnet, daß** das kationische Polymer unter Polyquaternium 5, Polyquaternium 6, Polyquaternium 7, Polyquaternium 11, Polyquaternium 15, Polyquaternium 16, Polyquaternium 22, Polyquaternium 28, Polyquaternium 39, Polyquaternium 44, Polyquaternium 46, Polyquaternium 47 und deren Gemischen ausgewählt ist.

11. Zusammensetzung nach einem der vorhergehenden Ansprüche, **dadurch gekennzeichnet, daß** der Mengenanteil des kationischen Polymers oder der kationischen Polymere im Bereich von 0,01 bis 5 Gew.-% Wirkstoff und vorzugsweise 0,05 bis 2 Gew.-% Wirkstoff, bezogen auf das Gesamtgewicht der Zusammensetzung, liegt.

12. Zusammensetzung nach einem der vorhergehenden Ansprüche, **dadurch gekennzeichnet, daß** sie enthält: (1) mindestens einen grenzflächenaktiven Stoff vom Phosphattyp, der unter Laurylmonophosphat, dem Kaliumsalz von Dodecylphosphorsäure, dem Monoester und Diester von Octylphosphorsäure, dem Phosphorsäuremonoester und Phosphorsäurediester von ethoxyliertem 2-Butyloctanol (7 mol EO), dem Kaliumsalz oder Triethanolaminsalz von Mono-C₁₂₋₁₃-alkylphosphat und Kaliumlaurylphosphat ausgewählt ist, (2) mindestens einen schäumenden nichtionischen grenzflächenaktiven Stoff, der unter den Alkylpolyglucosiden und den mehrfach mit Glycerin veretherten Fettalkoholen ausgewählt ist, und (3) mindestens ein kationisches Polymer enthält, das unter Polyquaternium 5, Polyquaternium 47, Polyquaternium 7, Polyquaternium 39, Polyquaternium 28 und Polyquaternium 44 ausgewählt ist.

13. Zusammensetzung nach einem der vorhergehenden Ansprüche, **dadurch gekennzeichnet, daß** sie mindestens ein Lösungsmittel enthält, das unter den niederen Alkoholen, Polyolen, Zuckern und deren Gemischen ausgewählt ist.

14. Zusammensetzung nach einem der vorhergehenden Ansprüche, **dadurch gekennzeichnet, daß** sie mindestens ein Verdickungsmittel enthält.

15. Kosmetische Verwendung einer Zusammensetzung nach einem der Ansprüche 1 bis 14 als Produkt zur Reinigung und/oder zum Abschminken der Haut, der Kopfhaut und/oder der Haare und/oder als abradierendes Produkt und/oder Exfoliationsmittel für die Haut.

16. Kosmetisches Verfahren zur Reinigung der Haut, der Kopfhaut und/oder der Haare, **dadurch gekennzeichnet, daß** die Zusammensetzung nach einem der Ansprüche 1 bis 14 in Gegenwart von Wasser auf die Haut, die Kopfhaut und/oder die Haare aufgebracht wird und der gebildete Schaum und die Schmutzrückstände durch Spülen mit Wasser entfernt werden.

17. Verwendung der Zusammensetzung nach einem der Ansprüche 1 bis 14 zur Herstellung einer Zusammensetzung zur Behandlung von fettiger Haut.
